# DEMANDE DE BREVET EUROPEEN

(11) **EP 1 611 850 A1**
(43) Date de publication de la demande: **04.01.2006**
(21) Numéro de dépôt: 04102983.6
(22) Date de dépôt: 28.06.2004
(51) Int. Cl.: A61B 17/00, A61B 17/10

(54) **Dispositif d'occlusion et de ponction étanche pour structure anatomique.**

(71) Demandeur: Cardio Life Research S.A., 1348 Louvain la Neuve (BE)
(72) Inventeur: DE CANIERE, Bernard, 1050, Bruxelles (BE); JOIE, Michel, 5030, Ernage (BE)
(74) Mandataire: Van Straaten, Joop

(57) **Abrégé**

Une agrafe chirurgicale (2) pour paroi (46) d'un organe cave, comprenant un dos (4) élastique et des ergots (10) disposés en deux rangées, en substance suivant des axes perpendiculaire au plan de la tête (4) élastique. En l'absence de sollicitations extérieure sur la tête (4), les extrémités libres des ergots (10) des deux rangées convergent. Lorsque le dos (4) est soumis à déformation, les axes des ergots (10) tendent à s'aligner parallèlement entre eux. L'agrafe (2) est munie d'une ouverture centrale (9) et de moyens d'accrochage (16) permettant de la maintenir en position déformée. Un dispositif de pose de l'agrafe (2) permet de réaliser une ponction sans écoulement de sang ou autre fluide dans un champ opératoire.

## Description

L'invention concerne un dispositif d'occlusion permettant une ponction ou une cannulation directe dans un organe creux, et notamment vasculaire, mais aussi dans des trachées, intestins, etc. Ce dispositif dispense de la nécessité d'une manoeuvre de fermeture chirurgicale lors du retrait du système de ponction ou de cannulation.

### Arrière-plan technologique de l'invention

Il est nécessaire, lors d'une intervention sur une artère, fût-elle aussi bénigne qu'une ponction ou une cannulation, de recourir à la suture de la membrane blessée, voire à l'apposition d'une pièce rapportée, comme décrit dans US-3 988 782.

US-2002/0082641 décrit une méthode et un dispositif pour la pose d'un clip vasculaire de forme plane correspondant au préambule de la revendication 1. Ce clip engendre toutefois une déformation accentuée (boursouflure) de la paroi à suturer et sa mise en place est relativement agressive.

La chirurgie cardiaque requiert le plus souvent un arrêt cardiaque afin d'obtenir un site opératoire immobile et exsangue permettant un geste chirurgical précis et délicat. Ceci nécessite le recours à la circulation extra-corporelle (CEC) afin de perfuser les organes systémiques (cerveau, foie, reins etc.) avec du sang oxygéné pendant la période où le coeur est arrêté.

Pour cela, il faut procéder au clampage de l'aorte, opération qui consiste le plus souvent à obturer le vaisseau par une pince externe qui est interposée entre la canule artérielle permettant la circulation extra-corporelle et l'orifice des artères coronaires. Cette manoeuvre isole la circulation coronaire du flux sanguin apporté par la CEC et donc permet d'arrêter le coeur.

L'injection d'une solution dans le réseau des artères coronaires (cardioplégie) protège le coeur lui-même pendant la période d'arrêt.

La mise en place de la circulation extra-corporelle (CEC), le clampage et la cardioplégie nécessitent classiquement la découpe et l'écartement du sternum (sternotomie). La sternotomie est une voie d'abord chirurgicale délabrante et porteuse de complications post opératoires fréquentes pour les patients.

Depuis plusieurs années, la chirurgie cardiaque développe des techniques alternatives visant à procurer une moindre agression pour les patients. L'éviction de la sternotomie est une de ces approches. Dans ce cas, l'intervention est conduite par des mini-incisions permettant l'introduction d'instruments endoscopiques.

Le but de l'invention est de permettre un branchement sur une structure anatomique sous pression, permettant de réaliser une ponction ou une cannulation sans épanchement de fluide et sans devoir refermer le trou de branchement par une suture.

Un autre but de l'invention est de pouvoir refermer une déchirure dans un organe cave, comme une artère, de façon durable et rapide.

L'objet de l'invention est une agrafe chirurgicale pour paroi d'un organe cave, comprenant un dos s'étendant sensiblement dans un plan, élastiquement ou plastiquement déformable, suivant les exigences,entre une position fermée de l'agrafe et une position ouverte de l'agrafe et au moins deux ergots espacés ; le dos comprend deux parties articulées l'une par rapport à l'autre ; les au moins deux ergots sont disposés en deux rangées sur ces deux parties en substance suivant des axes perpendiculaires au plan de chacune des deux parties ; en position fermée du dos, les extrémités libres des ergots des deux rangées convergent, lorsque le dos est en position ouverte, les axes des ergots des deux rangées tendent à s'aligner parallèlement entre eux et/ou sont séparés par une écartement plus important. La dite agrafe est munie d'une ouverture centrale et de moyens de préhension permettant de la maintenir en position.

L'avantage de l'invention est de permettre un branchement, notamment une ponction ou une cannulation, d'une artère sous pression comme l'aorte sans devoir la manipuler pour refermer le trou de ponction par une suture. Cette dernière manoeuvre est en effet potentiellement délétère : emboligène ou siège de déchirures du tissu artériel et d'hémorragies. Dans le cas présent, au contraire, les différentes couches de la paroi de l'artère sont maintenues pratiquement dans leurs positions relatives initiales, ce qui donne une cicatrisation rapide sans affaiblissement des propriétés de la paroi. Suivant une première forme de réalisation avantageuse le dos est élastiquement déformable.

Suivant une autre forme de réalisation avantageuse, le dos est plastiquement déformable.

Suivant une forme de réalisation avantageuse, les ergots sont rapportés sur le support déformable. Les ergots comprennent le cas échéant des barbelures.

Au moins le dos de l'agrafe est avantageusement réalisé en un matériau bio-compatible tel que les alliages de Titane et particulièrement le Nitinol.

De préférence, les deux parties supportant chacune une rangée d'ergots et un moyen de préhension, sont reliées par des jonctions élastiques ou plastiquement déformable, avantageusement en forme de zigzag.
Un autre objet de l'invention est un dispositif chirurgical de colmatage d'une incision dans la paroi d'un organe cave qui comprend
- une agrafe telle que décrit ci-dessus
- un moyen de maintien de la dite agrafe, le dit moyen étant apte à amener la dite agrafe de sa position ouverte à sa position fermée
- un support relié à un organe de traction s'étendant vers l'arrière, formant la tête du dispositif,
- un introducteur apte à introduire la tête du dispositif à l'intérieur d'un organisme.

Lorsque l'agrafe est élastiquement déformable, le moyen de maintien de la dite agrafe est de préférence une pince munie de deux mâchoires et d'un moyen de verrouillage ; l'extrémité libre des mâchoires est munie de moyens d'accrochage aptes à coopérer avec les moyens de préhension de l'agrafe ; des moyens de verrouillage maintiennent la dite agrafe sur les mâchoires en position déformée ; la dite agrafe sert de moyen de rappel tendant à rapprocher les dites mâchoires.

Lorsque l'agrafe est plastiquement déformable, le moyen de maintien de la dite agrafe est de préférence une pince munie de deux mâchoires et d'un moyen de verrouillage ; l'extrémité libre des mâchoires est munie de moyens d'accrochage aptes à coopérer avec les moyens de préhension de l'agrafe ; des moyens de verrouillage maintiennent la dite agrafe sur les mâchoires en position ouverte ; les mâchoires de la dite pince sont aptes à être actionnées par des moyens de serrage de façon à entraîner une déformation plastique du dos de l'agrafe.
Avantageusement, la tête du dispositif est détachable de l'introducteur. L'usage d'un introducteur détachable permet d'avoir recours à des conduits flexibles, ce qui dégage le champ opératoire.

Les moyens de verrouillage de la pince comprennent, de préférence, l'aiguille de ponction creuse elle-même. Il est ainsi impossible d'actionner le dispositif par erreur en cours d'opération. Au pire, ce retrait entraîne la fermeture « automatique » de la plaie.

Suivant un mode de réalisation particulier, l'introducteur comprend, du côté opposé à la tête, un organe de déverrouillage de la pince apte à opérer une traction sur le support d'aiguille creuse de façon à libérer l'agrafe. Cette disposition a l'avantage d'obliger l'opérateur à maintenir le dispositif contre l'organe creux au moment de la fermeture de l'agrafe, empêchant ainsi un faux positionnement de celle-ci.

Cet organe de déverrouillage peut être , au choix, actionné manuellement, électriquement ou pneumatiquement.

Suivant un mode de réalisation préféré, le support de l'aiguille creuse supporte en outre un dispositif de maintien , avantageusement une ventouse apte à s'appliquer contre la paroi de l'organe cave et à y maintenir en place la tête du dispositif, la dite ventouse étant reliée à un conduit apte à être raccordé à une source de pression négative.La présence d'un tel moyen de maintien permet à l'opérateur, une fois le dispositif en place, de se consacrer totalement au maniement des autres instruments chirurgicaux.

L'introducteur est, de façon avantageuse, plastiquement déformable de façon à pouvoir présenter l'aiguille perpendiculairement à une paroi se présentant sous un angle quelconque.

Suivant un mode de réalisation optionnel, le dispositif comprend un trocart insérable au travers de l'aiguille creuse, ce qui permet de donner à l'incision au travers de la paroi une forme plus nette.

Si l'on souhaite utiliser le dispositif uniquement dans sa fonction de fermeture de plaies, l'aiguille est remplacée par un simple embout ne dépassant pas les mâchoires de la pince.

### Brève description des Figures

D'autres particularités et avantages de l'invention ressortiront de la description ci-après de modes de réalisation particuliers de l'invention, référence étant faite aux figures annexées dans lesquelles :
La Fig. 1 est une vue en perspective d'une agrafe chirurgicale suivant l'invention, fortement agrandie, en l'absence de sollicitations extérieures.
La Fig. 2 est une vue en perspective de l'agrafe de la Fig. 1 soumise à un effort de torsion.
Les Fig. 3 à 9 se rapportent plus particulièrement à un dispositif de pose pour agrafe élastiquement déformable.

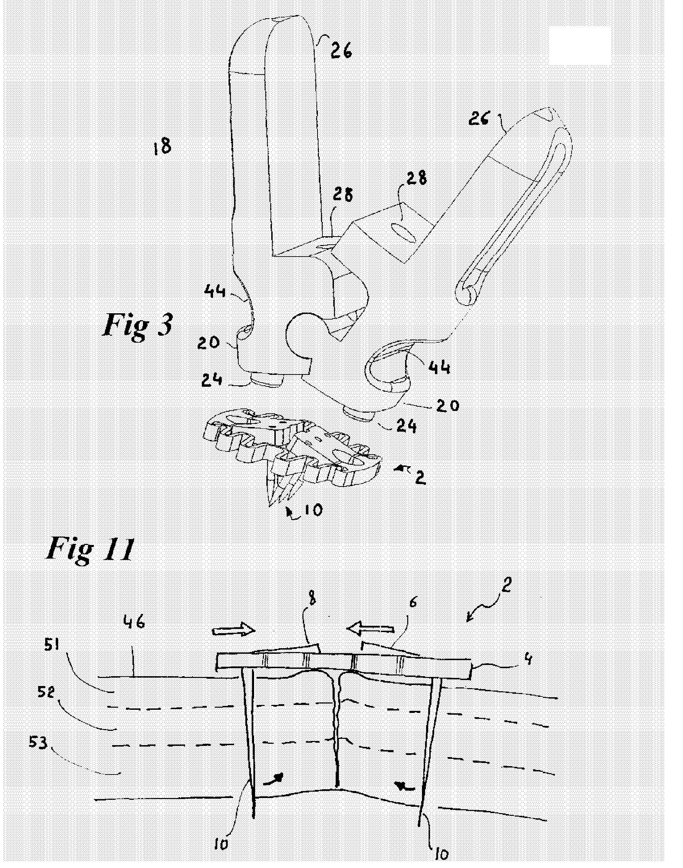

La Fig. 3 est une vue en perspective à une autre échelle de la pince du dispositif combiné de ponction suivant l'invention.

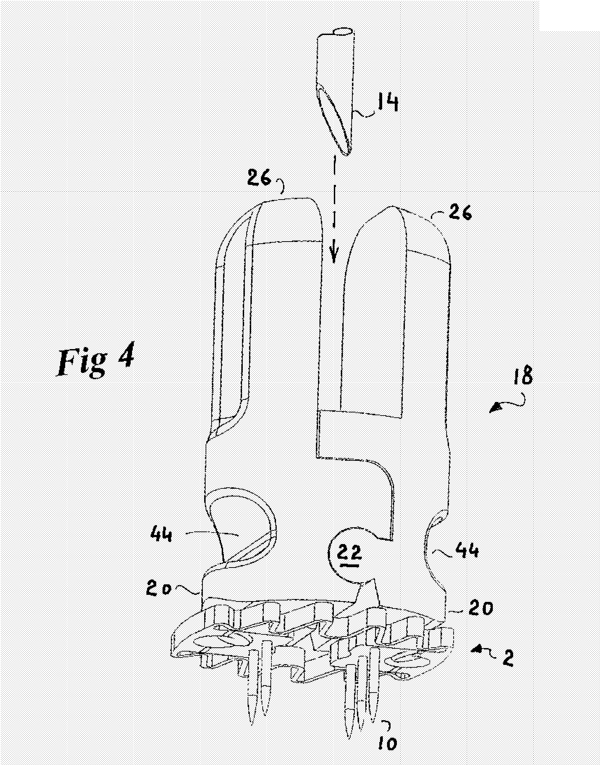

La Fig. 4 est une vue en perspective de la pince de la Fig. 3 en position fermée.

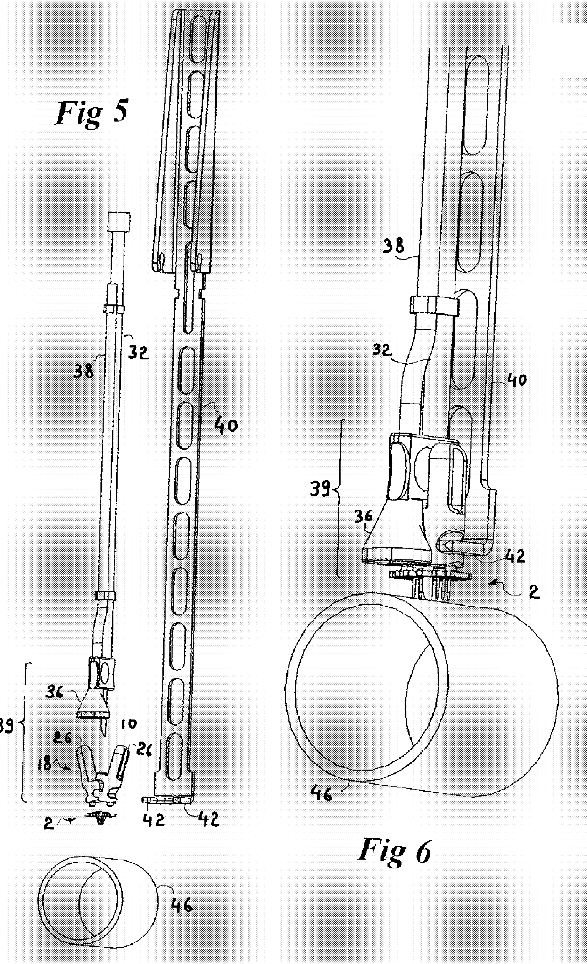

La Fig. 5 est une vue éclatée des différents constituants du dispositif combiné de ponction suivant l'invention.
La Fig. 6 est une vue en perspective de la tête du dispositif combiné au moment de sa mise en place sur une artère.

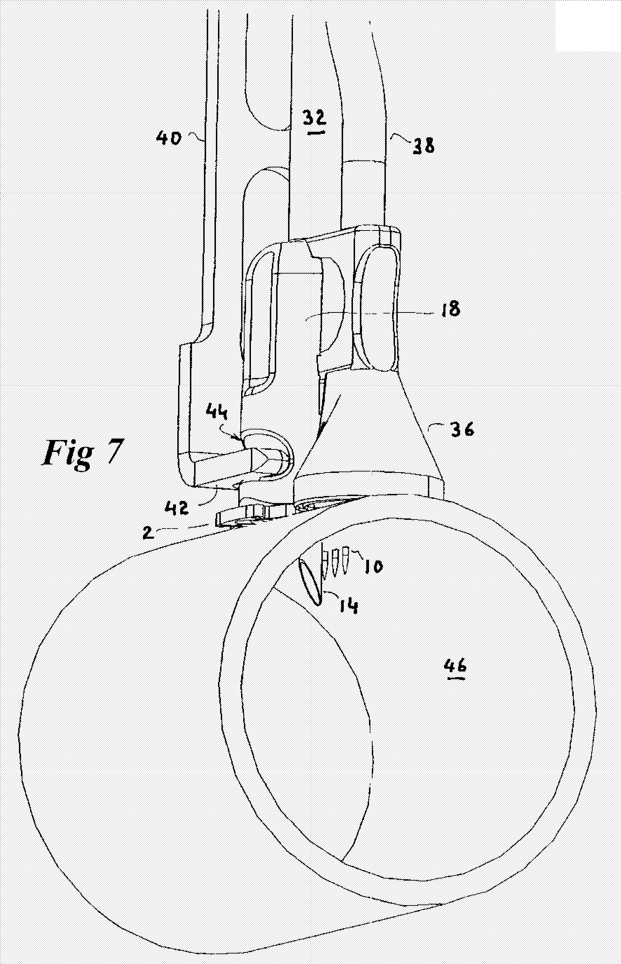

La Fig. 7 est une vue plus détaillée en perspective de la tête du dispositif après mise en place sur une artère.

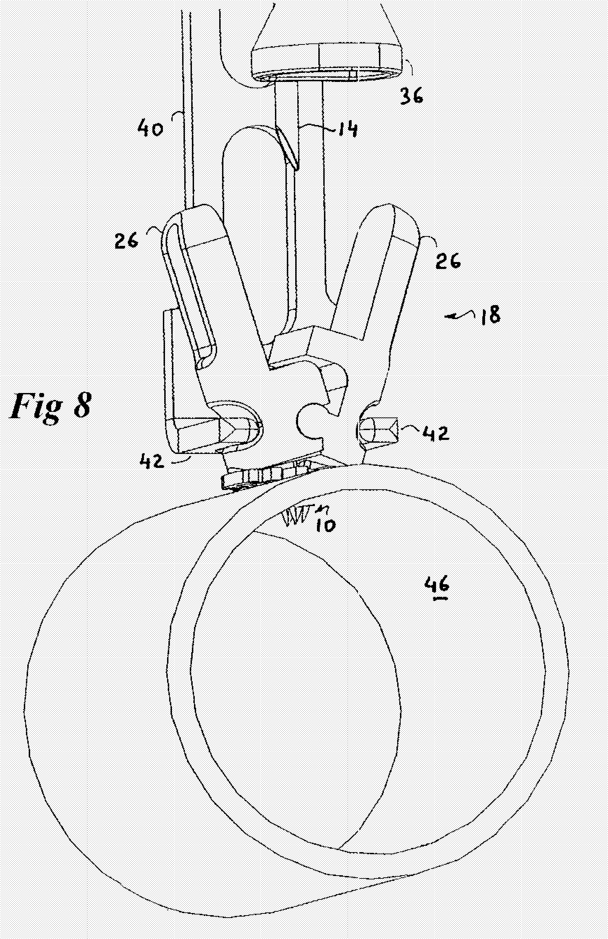

La Fig. 8 est une vue détaillée en perspective de la libération de l'agrafe suivant l'invention.

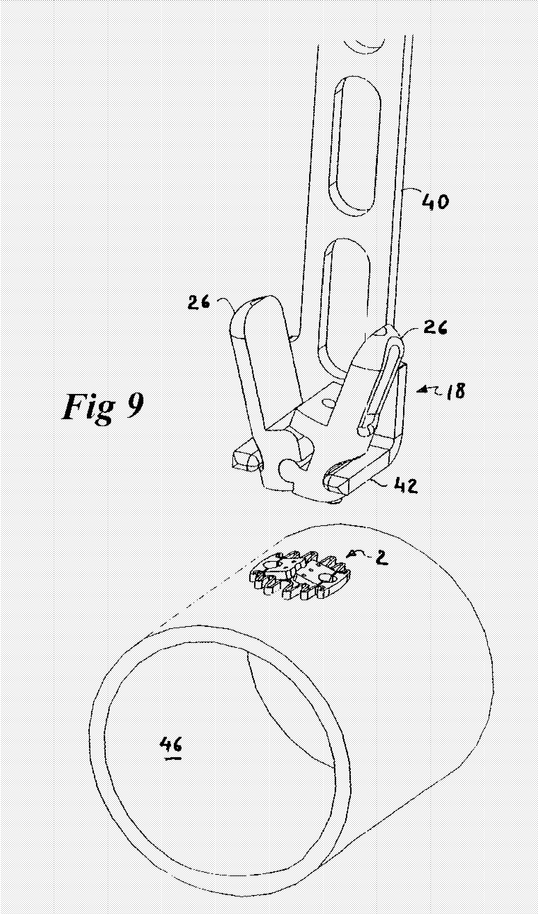

La Fig. 9 est une vue en perspective du retrait de la tête du dispositif après mise en place de l'agrafe.

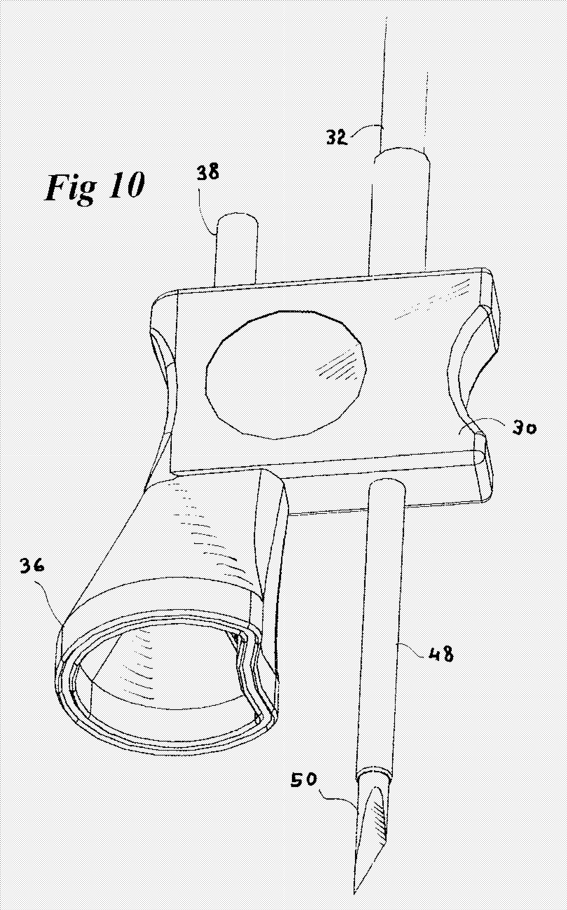

La Fig. 10 est une vue en perspective d'un autre mode de réalisation du dispositif suivant l'invention.

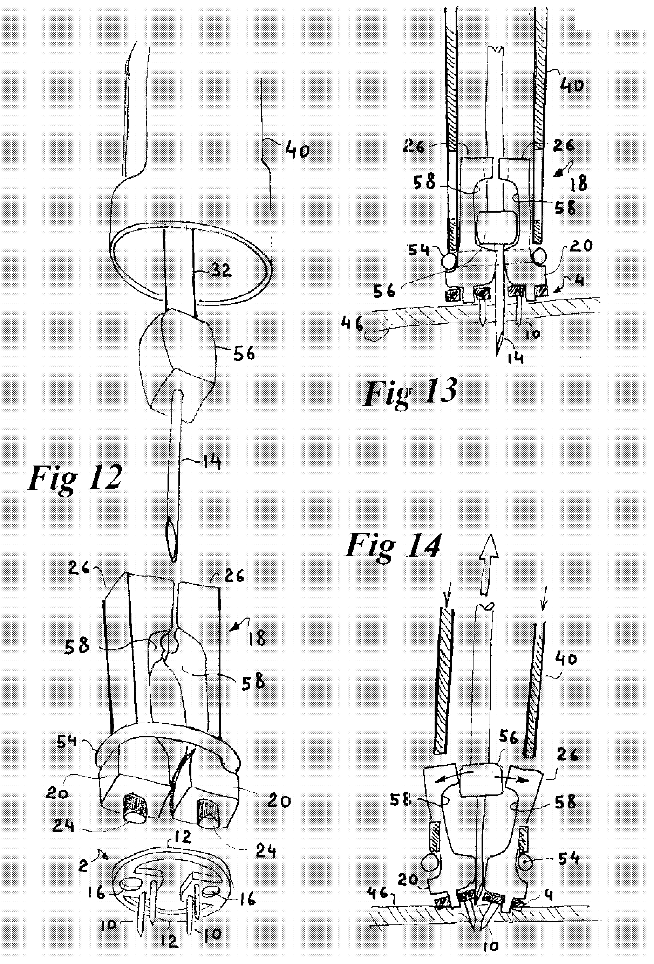

La Fig.11 est une vue schématique en coupe de l'agrafe en fonctionnement.
Les Fig 12 à 14 se rapportent plus particulièrement à un mode de réalisation du dispositif suivant l'invention muni d'une agrafe à déformation plastique.
La Fig. 12 est une vue éclatée de la tête d'un dispositif muni d'une agrafe à déformation plastique.
Les Fig 13 à 14 sont des vues schématiques en perspective des deux étapes de pose de l'agrafe avec le dispositif de la Fig. 12.

### Description détaillée des Figures

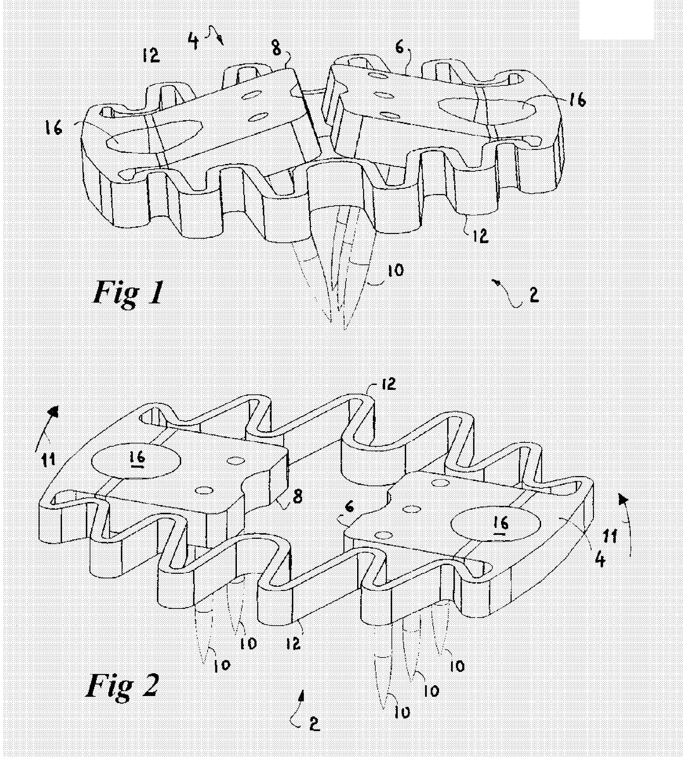

Les Fig. 1 et 2 montrent, fortement agrandie, une agrafe 2 suivant l'invention. Pour fixer un ordre d'idée, l'épaisseur du dos 4 de l'agrafe 2 représentée est de l'ordre du millimètre et la longueur des ergots de l'ordre de 4 mm. Le dos 4 comporte deux parties 6, 8 mobiles d'une par rapport à l'autre et supportant chacune une rangée d'ergots 10. Les ergots s'étendent, substantiellement, suivant un axe perpendiculaire au plan de chacune de ces parties 6,8 du dos 4. Ils sont , de préférence, disposés en quinconce de façon à assurer un pinçage régulier des tissus.

L'agrafe peut être réalisée soit en un matériau élastique (généralement métallique), soit en un matériau plastiquement déformable.

Dans le cas d'un matériau élastique, en l'absence de sollicitations extérieures, comme montré à la Fig. 1, les axes des ergots 10 des deux rangées convergent suivant un angle d'environ 30 à 40°. Ce point a son importance car, comme on le verra plus loin, il permet de refermer les lèvres d'une plaie sans perturber les positions relatives des différentes couches d'une paroi d'un organe.

Lorsqu'on l'on applique une torsion 11 au dos 4 (voir la Fig. 2), les axes des deux rangées d'ergots 10 s'alignent parallèlement à un axe en substance perpendiculaire au plan général du dos 4. Dans cette configuration, l'agrafe offre une résistance minimale à la pénétration dans les tissus. Des jonctions élastiques 12 en zigzag relient entre elles les deux parties 6, 8 du dos 4 de l'agrafe, ce qui permet, outre l'alignement des ergots, d'écarter les deux parties 6,8 l'une de l'autre. Les deux parties 6, 8 du dos 4 sont séparées par une ouverture centrale 9 qui permet d'insérer au travers du dos 4 et des deux rangées d'ergots 10 une aiguille de ponction 14 qui sera décrite en détail plus loin. Deux orifices 16 ménagés sur les deux parties 6, 8 servent de moyen d'accrochage de l'agrafe 2. Leur utilité deviendra apparente en se référant aux Fig. 3 et 4.

La Fig. 3 montre, toujours dans le cas d'une agrafe à dos élastique, une pince 18 munie de deux mâchoires 20 pivotant relativement à un axe commun 22. Chacune des mâchoires 20 est munie d'un plot de préhension 24 apte à coopérer avec les orifices 16 pour saisir l'agrafe 2. La pince étant en position ouverte, les plots 24 sont alignés de façon à permettre un engagement et un dégagement aisé de l'agrafe 2. En rapprochant les deux poignées 26 de la pince 18 (opération qui peut être réalisée manuellement juste avant une opération chirurgicale), on exerce sur les mâchoires 20 une force de traction qui, via les plots 24, amène l'agrafe 2 dans sa position sous contrainte, où les deux rangées d'ergots 10 ont des axes parallèles et où l'ouverture centrale 9 est distendue. La pince 18 étant en position fermée (comme représenté à la Fig. 4) les jonctions en zigzag 12 de l'agrafe 2, distendues, exercent sur les mâchoires 20 une force tendant à les rouvrir. Leur maintien en position fermée, et en conséquence l'assujettissement de l'agrafe 2 sur la pince 18 est assuré par un verrouillage. C'est l'aiguille de ponction 14 elle-même, enfilée au travers de deux lumières 28 traversant les poignées 26 des mâchoires 20, qui assure ici ce verrouillage.

Si l'on utilise une agrafe à dos déformable plastiquement, on utilisera une pince 18 de configuration légèrement différente, comme montré plus loin.

Les Fig.5 et 6 montrent, respectivement en éclaté et après assemblage, les différents éléments du dispositif de ponction combiné de l'invention.

L'aiguille de ponction 14 est insérée sur un support 30 auquel est raccordé un conduit 32 dont l'extrémité proximale est munie d'un connecteur 34 permettant de le brancher à une source de fluide tel que, par exemple, une solution de cardioplégie. On constate que le support d'aiguille 30 supporte latéralement un organe particulier : une ventouse 36 reliée par un second conduit 38 (courant parallèlement au conduit 32) à une pompe à vide.

La pince 18, l'aiguille 14 et son support 30 ainsi que la ventouse 36 forment la "tête" 39 du dispositif de ponction combiné de l'invention. Cette tête 39 est supportée par un introducteur 40, terminé ici par une "fourchette" , soit deux pointes 42 qui font saillie à son extrémité distale et qui, en s'insérant de part et d'autre de la pince 18 dans des rainures 44 prévues à cet effet, permettent de manipuler la tête 39, sans entraver le pivotement des mâchoires de la pince 18.

La succession des Fig. 6, 7, 8 et 9 permet de mieux comprendre tant le mode opératoire que les avantages du dispositif de l'invention.

A la Fig. 6, l'agrafe élastique 2 a été montée sur la pince 18 et celle-ci est maintenue en position fermée par l'aiguille 14. La tête 39 du dispositif supportant l'agrafe 2 est ensuite insérée sur l'introducteur 40. Le dispositif est introduit dans la cage thoracique d'un patient et la tête 39 est mise en place sur la paroi 46 d'un vaisseau sanguin ou d'un organe creux à ponctionner (par exemple, l'aorte). Le matériau de l'introducteur 40 étant ductile, l'opérateur a la faculté de le courber si la paroi 46 du vaisseau ne se présente pas sous l'angle adéquat. Il enfonce fermement les ergots 10 de l'agrafe 2 dans la paroi 46, leurs axes étant à ce moment disposés parallèlement à celui de l'aiguille 14 et/ou l'aiguille 14 elle-même(comme on le verra plus loin, il n'est pas indispensable que l'aiguille 14 soit insérée en même temps que les ergots 10 de l'agrafe 2). Les dimensions des ergots 10 ont été calculées en fonction de l'épaisseur de la paroi à traverser : une longueur suffisante pour assurer un ancrage ferme et un diamètre optimum pour éviter la déchirure des tissus au moment de la fermeture de l'agrafe 2. Un effet inattendu révélé lors des essais est que les perforations allongées associées à des ergots plus fins laissent passer moins de sang et se referment plus rapidement que des perforations rondes associées à des ergots de section plus large. Les ergots 10 comprennent éventuellement des barbelures.

L'opérateur fixe ensuite la tête du dispositif contre la paroi. En l'occurrence, il enclenche la mise en dépression de la ventouse 36, ce qui a pour conséquence de plaquer la tête 39 fermement contre la paroi 46 du vaisseau et de l'y maintenir. L'opérateur peut alors dégager la tête 39 de l'introducteur 40 par une translation latérale qui la libère des pointes 42 de la "fourchette". Les conduits 32 et 38 étant flexibles, l'opérateur peut les écarter du champ opératoire et poursuivre l'opération en cours sans s'en soucier davantage.

On notera que la ventouse 36 n'est pas le seul dispositif utilisable pour immobiliser le dispositif contre la paroi de l'artère : on peut également utiliser une bride ou une boucle en lasso entourant le périmètre de l'artère

On se trouve maintenant au stade montré à la Fig. 7. On peut soit opérer un prélèvement de sang dans l'aorte ou, au contraire, y injecter un produit quelconque via le conduit 32 et l'aiguille creuse 14.

A la Fig. 8, l'opération est en phase terminale : il n'est plus nécessaire, à ce moment, de maintenir en place l'aiguille de perfusion 14. L'opérateur remet en place l'introducteur 40 en insérant les pointes 42 de la "fourchette" dans les rainures 44 de la pince 18. Il saisit l'extrémité proximale 34 des conduits 32, 38 (à l'aide d'un dispositif non représenté, éventuellement solidaire de l'introducteur 40), interrompt la mise en dépression de la ventouse 36 et opère une traction sur le support 30.

L'aiguille 14, en se dégageant, déverrouille la pince 18 et libère l'agrafe 2. S'il s'agit d'une agrafe élastique, Sous l'action des jonctions en zigzag 12, les deux rangées d'ergots 10 de l'agrafe 2 se rapprochent et/ou convergent, pressant fermement l'une contre l'autre les lèvres de l'incision provoquée par l'aiguille 14 et empêchant tout épanchement de sang dans le champ opératoire. Ce mouvement de rapprochement est complété le cas échéant par la convergence des axes des ergots 10, ce qui a pour effet une retenue de l'agrafe 2 en position implantée.

Si l'on a utilisé une agrafe 2 à dos 4 plastiquement déformable, celle-ci n'exerce évidemment pas d'effet ressort sur la pince 18. L'agrafe 2 est mise en place au contraire en exerçant sur les branches de la pince une poussée, soit manuelle, soit par l'intermédiaire d'un moyen de contrainte (non représenté) mécanique , pneumatique ou électrique.

On procède ensuite au retrait de la pince 18, comme montré à la Fig. 9. L'agrafe 2, réalisée en un matériau bio-compatible bien toléré par l'organisme, reste en place. Vu sa géométrie, le cas échéant, elle peut même être retirée sans problème lors d'une opération ultérieure.

La Fig. 10 montre une autre forme de réalisation du dispositif de l'invention. Dans ce mode de réalisation, l'aiguille 14 est remplacée par une canule creuse 48 au travers duquel l'opérateur introduit un trocart 50, de façon à réaliser une incision plus nette (rectiligne) et mieux centrée par rapport aux rangées d'ergots 10 de l'agrafe 2 dans la paroi 46. La canule a l'avantage de permettre un débit optimal du fluide qui la traverse. Le recours au trocart 50 n'est évidemment possible que si l'introducteur est rectiligne ou sensiblement rectiligne. Par ailleurs, si l'on utilise une aiguille de ponction classique, on peut décaler la position relative des plots 24 ou des mâchoires 20 de la pince 18 par rapport à l'axe de l'aiguille 14 pour mieux centrer l'incision.

La Fig. 11 montre plus en détail l'action sur les tissus de l'agrafe 2. Comme on peut le constater, les différentes couches (couche externe 51, média 52, couches externe et interne de l'intima 53, etc) de la paroi sont maintenues pratiquement sans déformation par rapport à leur position initiale, ce qui favorise un cicatrisation sans problèmes. Comme on peut le voir, les ergots 10 ont une longueur en rapport avec l'épaisseur des tissus traversés, suffisante pour permettre un ancrage profond. Ils peuvent le cas échéant être munis de barbelures (non représentées).

On conçoit que l'agrafe 2, bien que représentée ici avec deux rangées d'un nombre limité d'ergots 10 (deux et trois, en l'occurrence), peut comprendre un nombre d'ergots différent(de 2 à N ergots), suivant les caractéristiques de la plaie à refermer. Il est avantageux que les ergots 10 des deux rangées soient disposés en quinconce, comme représenté, de façon à refermer la plaie sur toute sa longueur. Bien que le présent dispositif ait été présenté ici dans le cadre d'une opération de chirurgie mini-invasive, il peut être utilisé aussi en chirurgie courante.

Par ailleurs, Si l'on souhaite utiliser le dispositif uniquement dans sa fonction de fermeture de plaies, l'aiguille 14 est remplacée par un simple embout ne dépassant pas les mâchoires de la pince.

La Fig. 12 montre une autre forme de réalisation de la tête 39 du dispositif, adaptée à l'emploi d'une agrafe à dos déformable plastiquement. Dans ce cas, la pince 18 est munie d'un bracelet agissant comme moyen de verrouillage, qui maintient, en l'absence de sollicitations, les plots 24 de la pince dans une position telle que l'agrafe 2 puisse y être fixée à l'état ouvert (c'est-à-dire avec les ergots 10 en position sensiblement parallèle). Le support de l'aiguille 14 comprend un noyau 56 formant came, cependant que les faces internes des poignées 26 de la pince 18 comprennent chacune une rampe 58 apte à coopérer avec les faces externes du noyau 56 formant came.

Le dispositif en cours de ponction est représenté à la Fig. 13. La ponction étant terminée, l'opérateur exerce une traction relative sur l'aiguille 14, la tête du dispositif étant par ailleurs maintenue fermement contre la paroi (voir Fig. 14). Comme pour l'autre version du dispositif, cette traction est effectuée soit manuellement (par un système de levier, par exemple) soit pneumatiquement ou même électriquement.

Au passage, le noyau 56, agissant comme une came, force les poignée 26 de la pince 18 à s'écarter, déclanchant ainsi le serrage des mâchoires 20 et par conséquence la fermeture de l'agrafe 2 qui resserre les tissus ponctionnés.

## Revendications

1. une agrafe chirurgicale (2) pour paroi (46) d'un organe cave, comprenant un dos (4) déformable entre une position fermée de l'agrafe et une position ouverte de l'agrafe et au moins deux ergots (10) espacés
**caractérisée en ce que**
- le dos (4) comprend deux parties (6,8) articulées l'une par rapport à l'autre
- les au moins deux ergots (10) sont disposés en deux rangées sur ces deux parties (6,8), en substance suivant des axes perpendiculaires au plan de chacune des deux parties (6,8),
- en position fermée du dos (4), les extrémités libres des ergots (10) des deux rangées convergent,
- lorsque le dos (4) est en position ouverte, les axes des ergots (10) des deux rangées tendent à s'aligner parallèlement entre eux et/ou sont séparés par une écartement plus important,
la dite agrafe (2) étant munie d'une ouverture centrale (9) et de moyens de préhension (16) permettant de la maintenir en position.

2. une agrafe chirurgicale suivant la revendication 1, **caractérisée en ce que** le dos (4) est élastiquement déformable.

3. une agrafe chirurgicale suivant la revendication 1, **caractérisée en ce que** le dos (4) est plastiquement déformable.

4. une agrafe chirurgicale (2) suivant l'une quelconque des revendications 1 à 3, **caractérisée en ce que** au moins le dos (4) est réalisé en un matériau bio-compatible choisi parmi les alliages de titane.

5. une agrafe chirurgicale suivant l'une quelconque des revendications 2 et 4, **caractérisée en ce que** les deux parties (6,8) sont reliées par des jonctions (12) élastiques.

6. une agrafe chirurgicale suivant l'une quelconque des revendications précédentes, **caractérisée en ce que** les ergots (10) sont des éléments rapportés sur le dos (4).

7. une agrafe chirurgicale suivant l'une quelconque des revendications précédentes, **caractérisée en ce que** les ergots (10) comprennent des barbelures.

8. un dispositif chirurgical de colmatage d'une incision dans la paroi d'un organe cave
**caractérisé en ce qu'**il comprend
- une agrafe (2) suivant l'une quelconque des revendications 1 à 7,
- un moyen de maintien de la dite agrafe, le dit moyen étant apte à amener la dite agrafe de sa position ouverte à sa position fermée
- un support (30) relié à un organe de traction (32) s'étendant vers l'arrière, formant la tête (39) du dispositif,
- un introducteur (40) apte à introduire la tête (39) du dispositif à l'intérieur d'un organisme.

9. un dispositif chirurgical suivant la revendication 8, **caractérisé en ce que**, l'agrafe (2) étant élastiquement déformable, le moyen de maintien de la dite agrafe (2) est une pince (18) munie de deux mâchoires (20) et d'un moyen de verrouillage (28), l'extrémité libre des mâchoires (20) étant munie de moyens d'accrochage (24) aptes à coopérer avec les moyens de préhension (16) de l'agrafe (2), des moyens de verrouillage (14,28) maintenant la dite agrafe (2) sur les mâchoires (20) en position déformée, la dite agrafe (20) servant de moyen de rappel tendant à rapprocher les dites mâchoires (20).

10. un dispositif chirurgical suivant la revendication 8, **caractérisé en ce que**, l'agrafe étant plastiquement déformable, le moyen de maintien de la dite agrafe est une pince (18) munie de deux mâchoires (20) et d'un moyen de verrouillage (28), l'extrémité libre des mâchoires (20) étant munie de moyens d'accrochage (24) aptes à coopérer avec les moyens de préhension (16) de l'agrafe (2), des seconds moyens de verrouillage () maintenant la dite agrafe (2) sur les mâchoires (20) en position ouverte, les mâchoires de la dite pince étant aptes à être actionnées par des seconds moyens de serrage entraînant une déformation plastique du dos de l'agrafe (2)

11. un dispositif chirurgical suivant l'une quelconque des revendications 8 à 10, **caractérisé en ce qu'**il comprend une aiguille de ponction creuse (14), de diamètre correspondant à celui de l'ouverture centrale (9) de l'agrafe (2), la dite aiguille (14) étant montée sur un support (30) et reliée à un canal d'acheminement de fluide (32).

12. Un dispositif chirurgical suivant l'une quelconque des revendications 8 à 11 **caractérisé en ce que** la tête (39) du dispositif est détachable de l'introducteur (40).

13. Un dispositif chirurgical suivant l'une quelconque des revendications 11 à 12 **caractérisé en ce que** les moyens de verrouillage (14,28)de la pince (18) comprennent l'aiguille de ponction creuse (14) elle-même.

14. Un dispositif chirurgical suivant l'une quelconque des revendications 9 à 13 **caractérisé en ce que** l'introducteur (40) comprend, du côté opposé à la tête (39), un organe de déverrouillage de la pince(18) libérant l'agrafe (2).

15. Un dispositif chirurgical suivant l'une quelconque des revendications 8 à 14 **caractérisé en ce que** le support (30) comprend un moyen de maintien (36) apte à s'appliquer contre la paroi (46) d'un organe cave et à maintenir en place la tête (39) du dispositif pendant une ponction.

16. Un dispositif chirurgical suivant la revendications 15 **caractérisé en ce que** le moyen de maintien (36) comprend une ventouse (36), la dite ventouse (36) étant reliée à un conduit (38) apte à être relié à une source de pression négative.

17. Un dispositif chirurgical suivant l'une quelconque des revendications 8 à 16 **caractérisé en ce que** l'introducteur(40) est plastiquement déformable de façon à pouvoir présenter l'aiguille (14) perpendiculairement à une paroi (46) se présentant sous un angle quelconque.

18. Un dispositif chirurgical suivant l'une quelconque des revendications 11 à 17 **caractérisé en ce qu'**il comprend un trocart (50) insérable au travers de l'aiguille creuse.
